# EUROPÄISCHE PATENTANMELDUNG

(11) **EP 2 720 174 A1**
(43) Veröffentlichungstag der Anmeldung: **16.04.2014**
(21) Anmeldenummer: 12188132.0
(22) Anmeldetag: 11.10.2012
(51) Int. Cl.: G06K 19/067, G01N 33/02

(54) **Reifegradmessung von Kakaofrüchten**

(71) Anmelder: Luxin (Green Planet) AG, 6004 Luzern (CH)
(72) Erfinder: Burkhardt, Holger, 79777 Ühlingen-Birkendorf (DE); Glanzmann, Arthur, 6004 Luzern (CH)
(74) Vertreter: Rupp, Christian

(57) **Zusammenfassung**

Eine Reifegrad-Bestimmungs-Vorrichtung (1) beinhaltet eine Sensor-Einheit (2), welche ausgebildet ist, um Daten eines landwirtschaftlichen Produkts wie Kakaofrüchte, welche auf einen Reifegrad des landwirtschaftlichen Produkts schließen lassen zu bestimmen und eine Verarbeitungs-Einrichtung (50), welche ausgebildet ist, um anhand der Daten der Sensor-Einheit (2) den Reifegrad des landwirtschaftlichen Produkts vor der Ernte des landwirtschaftlichen Produkts zu ermitteln.

## Beschreibung

Die Erfindung betrifft eine Reifegrad-Bestimmungs-Vorrichtung, ein Reifegrad-Bestimmungs-System und ein Verfahren zur Reifegrad-Bestimmung von landwirtschaftlichen Produkten, insbesondere von Kakaofrüchten.

Es ist bei Kakaofrüchten besonders schwierig, den Reifegrad einer einzelnen Frucht von außen festzustellen. Dies liegt daran, da der immer grüne Kakaobaum in Regel ganzjährig blüht und folglich auch ganzjährig Früchte trägt. Somit befinden sich in der Regel in einem Kakaobaum Kakaofrüchte unterschiedlichen Reifegrades. Auch lässt sich der Reifegrad der Kakaofrucht bzw. der darin befindlichen Samen, der Kakaobohnen nur schwer an dessen Farbe erkennen. Die Bestimmung des Reifegrads von Kakaofrüchten erfordert somit viel Erfahrung, die in der Regel lediglich Vorarbeiter einer Kakaofarm besitzen. Dieser prüft die von den Erntekräften geernteten Früchte auf ihren Reifegrad und sortiert unreife Früchte aus. Regelmäßig kommt es dabei zu einem Ausschuss von über 10 %. Dies wirkt sich negativ auf die Ausbeute aus, sofern die unreifen Früchte hinreichend aussortiert werden. Ist dies nicht der Fall, führen die eingemischten unreifen Kakaobohnen zu einer Minderung der Kakaoqualität.

Die Reifegradbestimmung ist darüber hinaus besonders schwierig, da die bis zu 30 cm langen und von dem Kakaobaum herabhängenden Früchte vertikal von oben nach unten reifen. Somit muss für den optimalen Erntezeitpunkt der kurze Zeitraum abgepasst werden, in welchem die gesamte Frucht reif ist und nicht der untere Teil der Frucht noch unreif oder der obere Teil der Frucht schon überreif ist.

Darüber hinaus werden gerade technische Verfahren zur Ermittlung des Reifegrads bereits geernteter Früchte entwickelt. So zeigt der Artikel "Jäger des guten Geschmacks", Manfred Schäfers, Frankfurter Allgemeine Zeitung, 30. April 2012, ein Verfahren zur Ermittlung des Reifegrads von Mangos mittels eines chemisch sensitiven Haftetiketts, welches auf von reifen Mangos abgegebene Substanzen reagiert.

Das dort gezeigte Verfahren ist nachteilig, da es lediglich bei bereits geernteten Früchten einsetzbar ist. Darüber hinaus gibt nicht jedes landwirtschaftliche Produkt charakteristische Substanzen ab, welche auf seinen Reifegrad schließen lassen.

Der Erfindung liegt die Aufgabe zugrunde, eine Vorrichtung, ein Verfahren und ein System zu schaffen, welche eine Ermittlung eines Reifegrads unterschiedlicher landwirtschaftlicher Produkte mit geringem Aufwand bereits vor der Ernte ermöglichen.

Die Aufgabe wird erfindungsgemäß für die Vorrichtung durch die Merkmale des unabhängigen Anspruchs 1, für das System durch die Merkmale des unabhängigen Anspruchs 10 und für das Verfahren durch die Merkmale des unabhängigen Anspruchs 15 gelöst. Vorteilhafte Weiterbildungen sind Gegenstand der hierauf rückbezogenen Unteransprüche.

Die Erfindung befasst sich somit mit einer einfachen und schnellen sowie sicheren Methode zur Bestimmung des

Reifegrads von landwirtschaftlichen Produkten, insbesondere Kakaofrüchten. Eine dafür vorgesehene Vorrichtung weist vorzugsweise zumindest eine Klemme und wenigsten zwei Kontakte auf, welche diametral an gegenüberliegenden Seiten der Kakaofrucht angesetzt werden können. Die Vorrichtung macht sich dabei zu Nutze, dass bei steigendem Fruktosegehalt in der Frucht die elektrische Leitfähigkeit durch die Frucht erhöht wird. Anhand des Fruktosegehalts lässt sich dabei der Reifegrad bestimmen. Somit kann aufgrund der gemessenen Leitfähigkeit der Frucht über die anzulegenden Kontakte der Reifegrad an der dafür vorgesehenen Stelle exakt gemessen werden. Die elektrische Leitfähigkeit zur Bestimmung des Reifegrads lässt sich in vorgegebenen Wertbereichen festlegen. Somit kann durch jede Person auf einfache Weise der exakte Reifezustand einer Kakaofrucht bestimmt werden. Dies ist ohne großen technischen oder zeitlichen Aufwand sowie ohne Vorkenntnisse bezüglich der Kakaoernte möglich.

Eine erfindungsgemäße Reifegrad-Bestimmungs-Vorrichtung beinhaltet eine Sensor-Einheit, welche ausgebildet ist, um Daten eines landwirtschaftlichen Produkts, welche auf einen Reifegrad des landwirtschaftlichen Produkts schließen lassen zu bestimmen und eine Verarbeitungs-Einrichtung, welche ausgebildet ist, um anhand der Daten der Sensor-Einheit den Reifegrad des landwirtschaftlichen Produkts bevorzugt vor der Ernte des landwirtschaftlichen Produkts zu ermitteln. So ist es möglich, die Ernte erst vorzunehmen, wenn das Produkt seinen optimalen Reifezeitpunkt erreicht hat.

Vorzugsweise beinhaltet die Reifegrad-Bestimmungs-Vorrichtung eine Kalibrier-Einrichtung, welche ausgebildet ist, um eine Kalibrierung der Reifegrad-Bestimmungs-Vorrichtung für unterschiedliche landwirtschaftliche Produkte durchzuführen. So kann die Vorrichtung in landwirtschaftlichen Produktionsbetrieben, welche verschiedene Produkte anbauen für unterschiedliche Produkte eingesetzt werden.

Bevorzugt beinhaltet die Sensor-Einheit einen Zuckergehalt-Sensor, insbesondere ein Leitfähigkeits-Sensor, und/oder einen Säuregehalt-Sensor, und/oder einen Größensensor und/oder einen Farb-Sensor. So kann der Reifegrad für eine große Zahl verschiedener Produkte zuverlässig bestimmt werden.

Die Sensor-Einheit ist vorzugsweise ausgebildet, um Daten des landwirtschaftlichen Produkts, welche auf einen örtlich begrenzten teilweisen Reifegrad des landwirtschaftlichen Produkts schließen lassen zu bestimmen. Die Verarbeitungs-Einrichtung ist dann ausgebildet, um anhand der Daten der Sensor-Einheit den örtlich begrenzten teilweisen Reifegrad des landwirtschaftlichen Produkts zu ermitteln. So kann ein Optimaler Erntezeitpunkt für Produkte, welche örtlich unterschiedliche Reifegrade aufweisen bestimmt werden.

Vorzugsweise weist die Reifegrad-Bestimmungs-Vorrichtung weiterhin eine Ortsbestimmungs-Einrichtung auf, welche ausgebildet ist, um den geographischen Ort des landwirtschaftlichen Produkts zu ermitteln. Besonders bevorzugt ist die Ortsbestimmungs-Einrichtung weiterhin ausgebildet, um die Höhe des landwirtschaftlichen Produkts über dem Erdboden zu bestimmen. So kann für einen späteren Erntevorgang genau bestimmt werden, welche Produkte, z.B. Früchte an einem Strauch welchen Reifegrad aufweisen. Bevorzugt ist die Verarbeitungs-Einrichtung ausgebildet, um anhand der Daten der Sensor-Einheit einen optimalen Erntezeitpunkt des landwirtschaftlichen Produkts zu ermitteln. So kann mit nur einem Arbeitsdurchgang, d.h. einer Reifegradmessung an jedem Produkt der Zeitpunkt des Erntevorgangs festgelegt werden. Dies ermöglicht einen sehr arbeitssparenden Ablauf.

Vorzugsweise weist die Reifegrad-Bestimmungs-Vorrichtung weiterhin eine Markierungs-Einrichtung auf, welche ausgebildet ist, um das landwirtschaftliche Produkt und/oder einen Bereich in der unmittelbaren Umgebung des landwirtschaftlichen Produkts derart mit einer Markierung zu versehen, dass der Reifegrad des landwirtschaftlichen Produkts und/oder ein optimaler Erntezeitpunkt aus der Markierung hervorgehen. So kann nach Feststellung des Reifegrads bzw. des optimalen Erntezeitpunkts ohne eine weitere Messung die Ernte von ungelernten Kräften durchgeführt werden. Diesen stehen dabei die ermittelten Informationen zur Verfügung.

Die Markierungs-Einrichtung ist bevorzugt ausgebildet, um eine Farbmarke und/oder eine Infrarot-Farbmarke und/oder eine Ultraviolett-Farbmarke und/oder eine chemischen Marke und/oder ein Ritzmuster und/oder eine Stanzung und/oder einen drahtlos auslesbaren Mikrochip, insbesondere ein RFID-Chip, auf das landwirtschaftliche Produkt und/oder den Bereich in der unmittelbaren Umgebung des landwirtschaftlichen Produkts aufzubringen. So kann eine zuverlässige, haltbare und für den Verkauf unproblematische Markierung je nach Produkt gewählt werden.

Bevorzugt weist die Reifegrad-Bestimmungs-Vorrichtung weiterhin eine Übertragungs-Einrichtung auf, welche ausgebildet ist, um sämtliche durch die Reifegrad-Bestimmungs-Vorrichtung ermittelte Daten an eine Verarbeitungs-Einrichtung zu übertragen. So können die Daten in einem zentralen Steuer-Prozess genutzt werden. Auch eine statistische Auswertung wird so möglich.

Ein erfindungsgemäßes Reifegrad-Bestimmungs-System umfasst zumindest eine Reifegrad-Bestimmungs-Vorrichtung nach einem der Ansprüche 1 bis 9, eine Verarbeitungs-Einrichtung und eine Datenbank. Die Reifegrad-Bestimmungs-Vorrichtung ist dann ausgebildet, um ermittelte Daten an die Verarbeitungs-Einrichtung zu übertragen. Die Verarbeitungs-Einrichtung ist dann ausgebildet, um die übermittelten Daten in der Datenbank zu speichern. So können die ermittelten Daten zentral genutzt werden.

Vorzugsweise weist das Reifegrad-Bestimmungs-System weiterhin eine Anzeige-Einrichtung auf. Die Verarbeitungs-Einrichtung ist dann ausgebildet, um in der Datenbank gespeicherte Daten bezüglich des Reifegrads zumindest eines landwirtschaftlichen Produkts auf der Anzeige-Einrichtung grafisch darzustellen. So ist ein einfaches und übersichtliches Informieren des Nutzers möglich.

Die Reifegrad-Bestimmungs-Vorrichtung ist bevorzugt eine Reifegrad-Bestimmungs-Vorrichtung nach Anspruch 5. Die Verarbeitungs-Einrichtung ist dann ausgebildet, um in der Datenbank gespeicherte Daten bezüglich des Reifegrads zumindest eines landwirtschaftlichen Produkts gemeinsam mit dem geografischen Ort des landwirtschaftlichen Produkts und vorzugsweise gemeinsam mit der Höhe über dem Erdboden des landwirtschaftlichen Produkts, vorzugsweise als Kartendarstellung, mittels der Anzeige-Einrichtung grafisch darzustellen. So kann sich der Nutzer mit einem Blick über den Reifezustand der Produkte in dem gesamten Betrieb informieren.

Das Reifegrad-Bestimmungs-System weist bevorzugt weiterhin eine Steuereinrichtung auf, welche ausgebildet ist, um zumindest ein weiteres damit verbundenes landwirtschaftliches Gerät zu steuern. Das weitere landwirtschaftliche Gerät ist bevorzugt eine automatisierte Erntemaschine und/oder eine Bewässerungsanlage und/oder eine automatisierte Schädlingsbekämpfungsanlage und/oder eine automatisierte Düngungsanlage. So können Arbeitsabläufe optimiert und eingespart werden.

Vorzugsweise weist das Reifegrad-Bestimmungs-System weiterhin eine Ernteergebnis-Eingabe-Einrichtung auf, welche ausgebildet ist, um nach einer Ernte Daten, das Ernteergebnis betreffend, einzugeben. Die Verarbeitungs-Einrichtung ist dann ausgebildet, um eine Kalibrierung des Reifegrad-Bestimmungs-Systems basierend auf dem eingegebenen Ernteergebnis und auf dem ermittelten Reifegrad zum Zeitpunkt der Ernte durchzuführen. So können die Vorhersagen des optimalen Erntezeitpunkts weiter verbessert werden.

Ein erfindungsgemäßes Verfahren dient der Bestimmung eines Reifegrads eines landwirtschaftlichen Produkts. Es beinhaltet die folgenden Schritte beinhaltet:
- Bestimmung von Daten, welche auf den Reifegrad des landwirtschaftlichen Produkts schließen lassen, vor der Ernte des landwirtschaftlichen Produkts, und
- Bestimmen des Reifegrads basierend auf den Daten, welche auf den Reifegrad des landwirtschaftlichen Produkts schließen lassen. So ist es möglich, die Ernte erst vorzunehmen, wenn das Produkt seinen optimalen Reifezeitpunkt erreicht hat.

Nachfolgend wird die Erfindung anhand der Zeichnung, in der ein fortlaufendes Ausführungsbeispiel der Erfindung dargestellt ist, beispielhaft beschrieben. In der Zeichnung zeigen:
- Fig. 1: ein erstes Ausführungsbeispiel der erfindungsgemäßen Reifegrad-Bestimmungs-Vorrichtung;
- Fig. 2: eine Detailansicht eines zweiten Ausführungsbeispiels der erfindungsgemäßen Reifegrad-Bestimmungs-Vorrichtung;
- Fig. 3: ein drittes Ausführungsbeispiel der erfindungsgemäßen Reifegrad-Bestimmungs-Vorrichtung, dargestellt als Blockschaltbild;
- Fig. 4: ein Ausführungsbeispiel des erfindungsgemäßen Reifegrad-Bestimmungs-Systems dargestellt als Blockschaltbild, und
- Fig. 5: ein Ausführungsbeispiel des erfindungsgemäßen Verfahrens zur Bestimmung des Reifegrads eines landwirtschaftlichen Produkts.

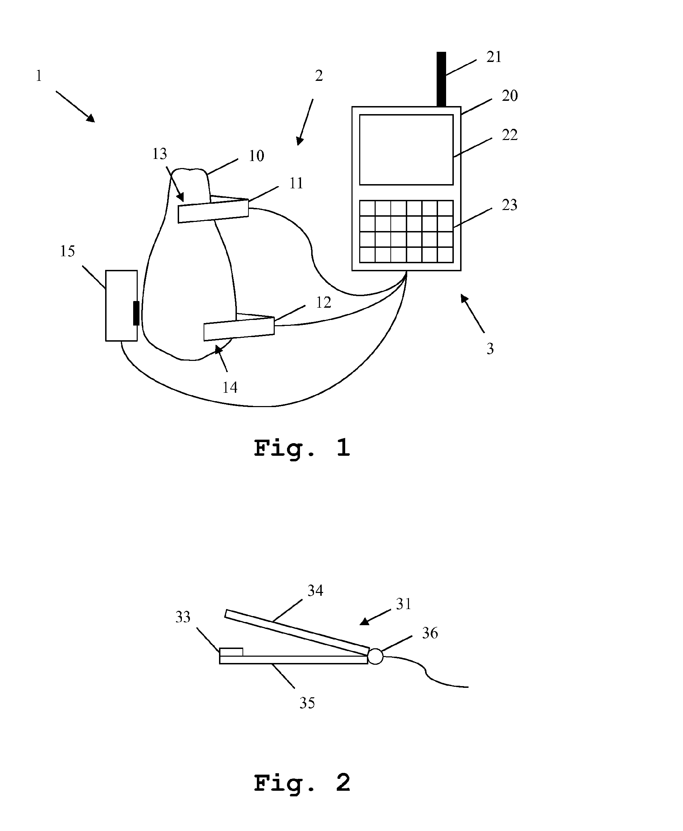

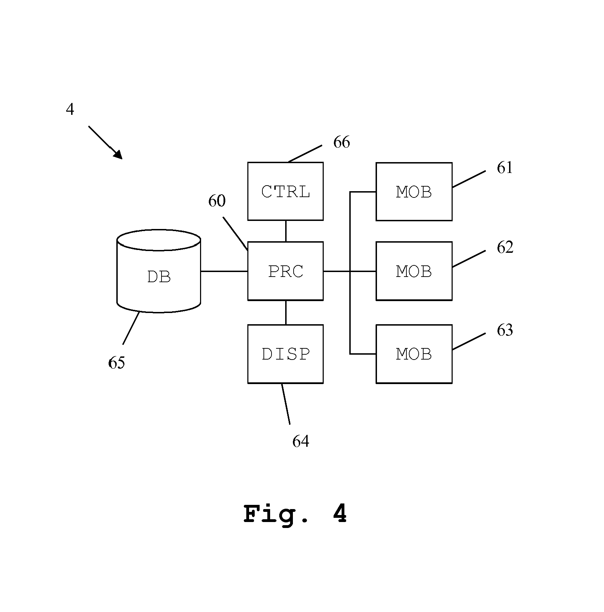

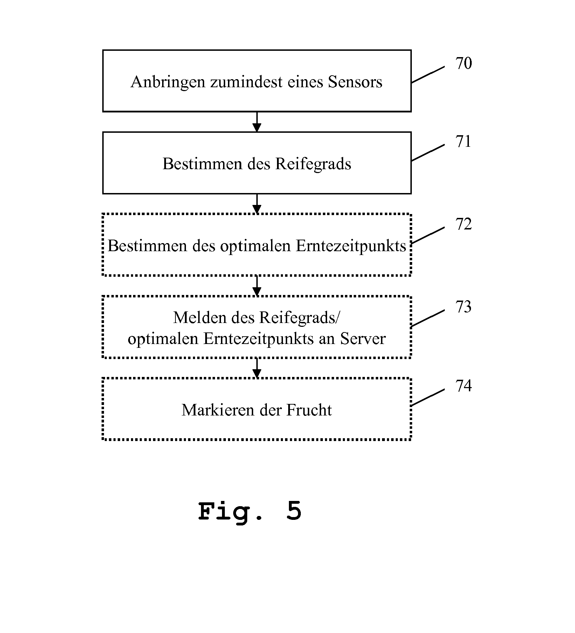

Zunächst wird anhand der Fig. 1-3 anhand mehrerer Ausführungsbeispiele der Aufbau und die Funktionsweise der erfindungsgemäßen Reifegradbestimmungsvorrichtung erläutert. Mittels Fig. 4 wird anschließend anhand eines Ausführungsbeispiels auf den Aufbau und die Funktionsweise des erfindungsgemäßen Reifegrad-Bestimmungs-Systems eingegangen. Abschließend wird anhand von Fig. 5 der genaue Ablauf und die genaue Funktionsweise eines Ausführungsbeispiels des erfindungsgemäßen Verfahrens zur Bestimmung des Reifegrads bezeigt. Identische Elemente wurden in ähnlichen Abbildungen zum Teil nicht wiederholt dargestellt und beschrieben.

In Fig. 1 ist ein erstes Ausführungsbeispiel der erfindungsgemäßen Reifegrad-Bestimmungs-Vorrichtung 1 gezeigt. Sie besteht aus einer Sensor-Einheit 2 oder einem Handgerät 3. Das landwirtschaftliche Produkt, hier eine Kakaofrucht 10 ist in zwei Klemmen 11, 12 eingespannt. Die Kakaofrucht 10 ist hier einzeln dargestellt. In der Praxis ist sie jedoch zum Zeitpunkt der Nutzung der Reifegrad-Bestimmungs-Vorrichtung 1 nach wie vor am Kakao-Strauch festgewachsen. Der Strauch ist hier lediglich der Übersichtlichkeit halber nicht dargestellt.

Die Klemmen 11, 12 beinhalten dabei jeweils einen Sensor 13, 14, welcher hier aufgrund der Verdeckung nicht dargestellt ist. Die Position der Sensoren ist jedoch mit den Bezugszeichen 13, 14 angedeutet. Die Sensoren 13, 14 befinden sich dabei jeweils an den Innenseiten der Klemmen 11, 12. Die Klemmen 11, 12 sorgen dabei dafür, dass die Sensoren 13, 14 eine geeignete Position an der Kakaofrucht 10 einnehmen und nicht verrutschen. Die Klemmen 11, 12 sind mittels Leitungen mit dem Handgerät 3 verbunden. Alternativ könnten die Klemmen 11, 12 auch über eine drahtlose Verbindung mit dem Handgerät 3 verbunden sein. In diesem Fall verfügen die Klemmen über jeweils eine Schnittelleneinheit, welche die drahtlose Verbindung herstellt. Der genaue Aufbau der Klemmen 11, 12 wird anhand von Fig. 2 näher erläutert.

Bei den Sensoren 13, 14 handelt es sich um einen Zuckergehalt-Sensor, insbesondere einen Leitfähigkeits-Sensor oder einen Säuregehalt-Sensor, oder einen GrößenSensor, oder einen Farb-Sensor.

Über die zwei hier dargestellten Klemmen 11, 12 hinaus können selbstverständlich weitere Klemmen auf der Länge der Kakaofrucht 10 angeordnet werden. Hierdurch kann ein Reifegrad individueller Bereiche der Kakaofrucht 10 ermittelt werden.

Weiterhin verfügt die Sensor-Einheit 2 über eine Markierungs-Einrichtung 15, welche hier neben der Kakaofrucht 10 angeordnet ist. Die Markierungs-Einrichtung 15 ist dabei ausgebildet, um die Kakaofrucht 10 mit einer Markierung zu versehen. Die Markierung dient dabei der Darstellung des aktuellen Reifegrads oder eines aus dem aktuellen Reifegrad ermittelten optimalen Erntezeitpunkt. Die Markierungs-Einheit 15 kann dabei die Kakaofrucht 10 mittels einer Farbmarkierung, oder einer IR-Farbmarkierung oder einer UV-Farbmarkierung, oder einem drahtlos auslesbaren Mikrochip, einem RFID, oder einer chemischen Marke, oder einem Ritzmuster, oder einer Stanzung als Markierung versehen. Die Markierungs-Einrichtung 15 ist dabei von dem Handgerät 3 gesteuert.

Das Handgerät 3 weist dabei ein Gehäuse 20 auf. In das Gehäuse 20 integriert ist ein Anzeige-Einrichtung 22, eine Bedieneinrichtung 23 und eine Antenne 21. Mittels der Anzeige-Einrichtung 22 und der Bedieneinrichtung 23 wird die Reifegrad-Bestimmungs-Vorrichtung 1 bedient. Dabei wird auf der Anzeige-Einrichtung 22 eine Nutzerführung dargestellt, in welche der Nutzer mittels der Bedieneinrichtung 23 Einstellungen vornehmen kann, den Messvorgang beginnen kann und eine Übertragung der ermittelten Daten veranlassen kann. Eine solche Datenübertragung erfolgt dabei über die Antenne 21. Diese ist ausgebildet, um eine Verbindung zu einer Steuer-Einrichtung eines Reifegrad-Bestimmungs-Systems herzustellen. Auf den Aufbau und die Funktionsweise eines erfindungsgemäßen Reifegrad-Bestimmungs-Systems wird anhand von Fig. 4 näher eingegangen.

Um nun den Reifegrad einer Kakaofrucht 10 zu bestimmen, legt der Nutzer die Klemmen 11 und 12 an bestimmte Positionen der Kakaofrucht 10 an. Die Sensoren 13, 14 werden dadurch in Position gegenüber der Kakaofrucht 10 gebracht. Mittels der Bedieneinrichtung 23 wählt der Nutzer auf der Anzeige-Einrichtung 22 den Start eines Messvorgangs aus. Das Handgerät 3 startet nun den Messvorgang. Die Sensoren 13, 14 werden somit dazu veranlasst, eine Messung durchzuführen und somit Messwerte aufzunehmen. Diese Messwerte werden an das Handgerät 3 übertragen und von diesem verarbeitet. Optional werden die ermittelten Messwerte mittels der Antenne 21 an das Reifegrad-Bestimmungs-System übertragen.

Weiterhin wird optional die Anbringung einer Markierung mittels der Markierungseinrichtung 15 bewirkt. Die Markierung wird dabei auf die Kakao-Frucht 1 oder in die Nähe der Kakao-Frucht, z.B. an dem Strauch oder dem Erdboden angebracht. Dies ist insbesondere dann relevant, wenn die Markierung später nicht auf der Frucht sichtbar sein soll. Insbesondere bei landwirtschaftlichen Produkten, welche mit Schale in den Handel kommen, wie z.B. Mangos ist dies relevant.

In Fig. 2 ist eine Detailansicht eines zweiten Ausführungsbeispiels der erfindungsgemäßen Reifegrad-Bestimmungs-Vorrichtung 1 gezeigt. Insbesondere ist hier eine Klemme 31 dargestellt. Die hier dargestellte Klemme 31 entspricht den Klemmen 11, 12 aus Fig. 1. Die Klemme 31 beinhaltet dabei eine erste Klemm-Backe 34 und eine zweite Klemm-Backe 35. Diese sind mittels eines Drehgelenks 36 miteinander verbunden. Vorzugsweise beinhaltet das Drehgelenk 36 einen Rückstellmechanismus, z.B. eine Feder, welcher die Klemm-Backen 34, 35 mit einer Klemmkraft versieht. An zumindest einer der Klemm-Backen 34, 35 ist zumindest ein Sensor 33 angebracht. Durch die Klemmkraft wird der Sensor 33 an die Kakaofrucht 10 gepresst.

Vorzugsweise können mehrere Sensoren an der Klemm-Backe 35 angeordnet sein. Alternativ können zusätzlich ein oder mehrere Sensoren an der ersten Klemm-Backe 34 angeordnet sein.

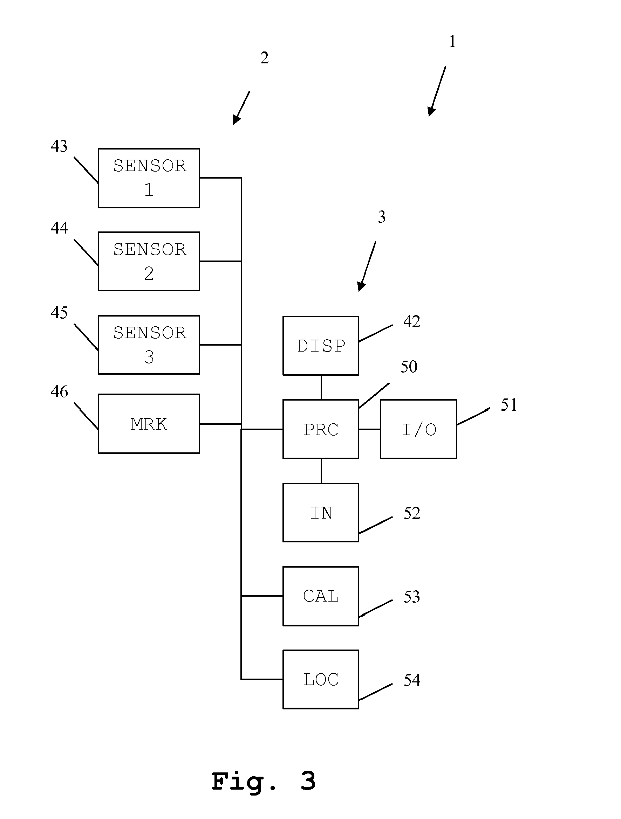

In Fig. 3 ist ein drittes Ausführungsbeispiel der erfindungsgemäßen Reifegrad-Bestimmungs-Vorrichtung 1 als Blockschaltbild dargestellt. Die Reifegrad-Bestimmungs-Vorrichtung 1 beinhaltet dabei eine Sensoreinheit 2, welche einen ersten Sensor 43, einen zweiten Sensor 44, einen dritten Sensor 45 und eine Markierungs-Einheit 46 beinhaltet. Weiterhin beinhaltet die Reifegrad-Bestimmungs-Vorrichtung 1 ein Handgerät 3, welches eine Verarbeitungs-Einrichtung 50 beinhaltet. Verbunden mit der Verarbeitungs-Einrichtung 50 sind eine Anzeige-Einrichtung 42 und eine Eingabe-Einrichtung 43. Darüber hinaus ist mit der Verarbeitungs-Einrichtung 50 eine SchnittstellenEinrichtung 51 verbunden, welche beispielsweise die Antenne 21 aus Fig. 1 beinhaltet. Darüber hinaus sind mit der Verarbeitungs-Einrichtung 50 eine Kalibrierungseinrichtung 53 und eine Ortsbestimmungseinrichtung 54 verbunden.

Die Sensoren 43-45 entsprechen dabei den Sensoren 13, 14 aus Fig. 1. Die Markierungs-Einrichtung 46 entspricht dabei der Markierungs-Einrichtung 15 aus Fig. 1. Die Eingabe-Einrichtung 52 entspricht dabei der Tastatur 23 aus Fig. 1. Die Anzeige-Einrichtung 42 entspricht dabei der Anzeige-Einrichtung 22 aus Fig. 1.

Die Verbindung der Sensor-Einheit 2 und des Handgeräts 3 erfolgt dabei ebenfalls über eine Verbindung der Verarbeitungs-Einrichtung 50 des Handgeräts 3 mit sämtlichen Komponenten der Sensor-Einheit 2. Ausdrücklich sei hier erwähnt, dass die Sensor-Einheit 2 ebenfalls mittels einer drahtlosen Verbindung mit der Verarbeitungs-Einrichtung 50 verbunden sein kann. Die drahtlose Verbindung erfolgt dabei optional über die Schnittstelleneinrichtung 51. Falls eine solche drahtlose Verbindung genutzt wird, verfügt die Sensor-Einheit 2 ebenfalls über eine entsprechende Schnittstelleneinheit.

Die Verarbeitungs-Einrichtung 50 steuert dabei sämtliche übrige Komponenten. Hinsichtlich der Funktion der einzelnen Elemente verweisen wir auf die Ausführungen zu Fig. 1. Darüber hinaus ermöglicht die Kalibrierungseinrichtung 53 eine Kalibrierung der Reifegrad-Bestimmungs-Vorrichtung 1 für unterschiedliche landwirtschaftliche Produkte. Auch innerhalb einer einzelnen Sorte kann z.B. eine Kalibrierung für unterschiedliche Schalendicken oder unterschiedliche Standorte durchgeführt werden.

Die Ortsbestimmungseinrichtung 54 dient der Bestimmung des gegenwärtigen Standorts der Reifegrad-Bestimmungs-Vorrichtung 1. Sie beinhaltet z.B. einen GPS-Empfänger. Um eine besonders genaue Ortsbestimmung individueller Früchte zu ermöglichen, kann die Ortsbestimmungseinrichtung 54 zusätzlich über einen Laserscanner verfügen, welcher einen Abstand einer gerade vermessenen Frucht zum Erdboden oder zu anderen Früchten oder zu einem Stamm oder anderen Ortsfesten Objekten ermittelt. Somit können auch mehrere übereinander angeordnete Früchte, welche identische GPS-Koordinaten aufweisen unterschieden werden.

In Fig. 4 ist ein Ausführungsbeispiel des erfindungsgemäßen Reifegrad-Bestimmungs-Systems 4 dargestellt. Das Reifegrad-Bestimmungs-System 4 beinhaltet mehrere Reifegrad-Bestimmungs-Vorrichtungen 61-63. Darüber hinaus beinhaltet es eine Verarbeitungs-Einrichtung 60, welche mit sämtlichen der Reifegrad-Bestimmungs-Vorrichtungen 61-63 verbunden ist. Die Verbindung ist hier als drahtgebundene Verbindung dargestellt. In der Praxis wird jedoch häufig eine drahtlose Verbindung mittels der Antenne 21 aus Fig. 1 eingesetzt. Zwischen die Verarbeitungs-Einrichtung 60 und die Reifegrad-Bestimmungs-Vorrichtungen 61-63 ist dann eine Schnittstelleneinrichtung zum Aufbau der drahtlosen Verbindung eingefügt.

Mit der Verarbeitungs-Einrichtung 60 sind weiterhin eine Anzeige-Einrichtung 64, eine Datenbank 65 und eine Steuer-Einrichtung 66 verbunden. Mittels der Anzeige-Einrichtung 64 können Ergebnisse der Reifegradbestimmung durch die Reifegrad-Bestimmungs-Vorrichtungen 61-63 angezeigt werden. Mittels der Datenbank 65 können die Ergebnisse der Messungen der Reifegrad-Bestimmungs-Vorrichtungen 61-63 und die geographischen Positionen, an welchen die Messungen durchgeführt wurden, gespeichert werden. Auch die Positionen der individuellen vermessenen Früchte können so gespeichert werden. In Zusammenarbeit mit der Anzeige-Einrichtung 64, der Datenbank 65 und der Verarbeitungs-Einrichtung 60 kann somit eine Kartendarstellung auf der Anzeige-Einrichtung 64 erzeugt werden, welche einen durchschnittlichen Reifegrad in einem Gebiet anzeigt, erzeugt werden.

Über die Steuer-Einrichtung 66 ist das Reifegrad-Bestimmungs-System 4 darüber hinaus in der Lage, weitere verbundene landwirtschaftliche Geräte zu bedienen. So ist z. B. eine Steuerung einer automatisierten Erntemaschine und/oder einer Bewässerungsanlage und/oder einer automatisierten Schädlingsbekämpfungsanlage und/oder einer automatisierten Düngungsanlage möglich. Diese weiteren landwirtschaftlichen Geräte sind dabei jedoch nicht Teil des Reifegrad-Bestimmungs-Systems. Unter Heranziehung der in der Datenbank 65 gespeicherten Messergebnisse ist somit eine optimale Steuerung der weiteren landwirtschaftlichen Geräte möglich. So kann eine optimale Ernte, eine optimale Düngung, eine optimale Schädlingsbekämpfung etc. erreicht werden.

Darüber hinaus ist die Verarbeitungs-Einrichtung 60 in der Lage mittels der in der Datenbank 65 gespeicherten Informationen Prognosen über die in Zukunft anfallenden Erntevolumina und einen zukünftig anfallenden Arbeitsbedarf treffen. Auch diese können über die Anzeige-Einrichtung 64 angezeigt werden.

Auch können in die Datenbank 65 nach erfolgter Ernte Informationen über die Ernteerträge bezogen auf die einzelnen Bereiche einer Plantage gespeichert werden. Basierend auf diesen Ernte-Informationen und den zuvor ermittelten Messwerten der Reifegrad-Bestimmungs-Vorrichtungen 61-63 kann eine Kalibrierung des Reifegrad-Bestimmungs-Systems erfolgen.

In Fig. 5 ist ein Ausführungsbeispiel des erfindungsgemäßen Verfahrens zur Bestimmung des Reifegrads gezeigt. In einem ersten Schritt 70 wird zumindest ein Sensor an einem landwirtschaftlichen Produkt, z.B. einer Kakaofrucht angebracht. In einem zweiten Schritt 71 wird mittels des zumindest einen Sensors ein Messwert ermittelt, welcher auf den Reifegrad schließen lässt. Aus dem Messwert wird anschließend der gegenwärtige Reifegrad bestimmt. In einem optionalen dritten Schritt 72 wird basierend auf dem gegenwärtigen Reifegrad ein optimaler Erntezeitpunkt bestimmt. In einem weiteren optionalen Schritt 73 wird der gegenwärtige Reifegrad bzw. Der optimale Erntezeitpunkt an einen zentralen Server übermittelt. Der zentrale Server entspricht hier der Verarbeitungs-Einrichtung 60, der Anzeige-Einrichtung 64, der Datenbank 65 und der Steuer-Einrichtung 66 aus Fig. 4. In einem fünften optionalen Schritt 74 erfolgt weiterhin eine Markierung des individuellen landwirtschaftlichen Produkts, z.B. der Kakaofrucht mit dem gegenwärtigen Reifegrad und/oder einem optimalen Erntezeitpunkt.

Die gegenwärtige Erfindung ist nicht auf die dargestellten Ausführungsbeispiele beschränkt. Insbesondere ist die gegenwärtige Erfindung für eine Vielzahl von landwirtschaftlichen Produkten einsetzbar. Auch können verschiedenste Sensoren zur Bestimmung des Reifegrads der unterschiedlichen landwirtschaftlichen Produkte eingesetzt werden. Alle vorstehend beschriebenen Merkmale oder in den Figuren gezeigten Merkmale sind im Rahmen der Erfindung beliebig vorteilhaft miteinander kombinierbar.

## Patentansprüche

1. Reifegrad-Bestimmungs-Vorrichtung (1) mit einer Sensor-Einheit (2), welche ausgebildet ist, um Daten eines landwirtschaftlichen Produkts (10), welche auf einen Reifegrad des landwirtschaftlichen Produkts (10) schließen lassen zu bestimmen, und
einer Verarbeitungs-Einrichtung (50), welche ausgebildet ist, um anhand der Daten der Sensor-Einheit (2) den Reifegrad des landwirtschaftlichen Produkts (10) bevorzugt vor der Ernte des landwirtschaftlichen Produkts (10) zu ermitteln.

2. Reifegrad-Bestimmungs-Vorrichtung (1) nach Anspruch 1,
**dadurch gekennzeichnet,**
**dass** die Reifegrad-Bestimmungs-Vorrichtung (1) eine Kalibrier-Einrichtung (53) beinhaltet, welche ausgebildet ist, um eine Kalibrierung der Reifegrad-Bestimmungs-Vorrichtung (1) für unterschiedliche landwirtschaftliche Produkte durchzuführen.

3. Reifegrad-Bestimmungs-Vorrichtung (1) nach Anspruch 1 oder 2,
**dadurch gekennzeichnet,**
**dass** die Sensor-Einheit (2) einen Zuckergehalt-Sensor, insbesondere ein Leitfähigkeits-Sensor, und/oder einen Säuregehalt-Sensor, und/oder einen Größensensor und/oder einen Farb-Sensor beinhaltet.

4. Reifegrad-Bestimmungs-Vorrichtung (1) nach einem der Ansprüche 1 bis 3,
**dadurch gekennzeichnet,**
**dass** die Sensor-Einheit (2) ausgebildet ist, um Daten des landwirtschaftlichen Produkts (10), welche auf einen örtlich begrenzten teilweisen Reifegrad des landwirtschaftlichen Produkts (10) schließen lassen zu bestimmen, und
**dass** die Verarbeitungs-Einrichtung (50) ausgebildet ist, um anhand der Daten der Sensor-Einheit (2) den örtlich begrenzten teilweisen Reifegrad des landwirtschaftlichen Produkts (10) zu ermitteln.

5. Reifegrad-Bestimmungs-Vorrichtung (1) nach einem der Ansprüche 1 bis 4,
**dadurch gekennzeichnet,**
**dass** die Reifegrad-Bestimmungs-Vorrichtung (1) weiterhin eine Ortsbestimmungs-Einrichtung (54) aufweist, welche ausgebildet ist, um den geographischen Ort des landwirtschaftlichen Produkts (10) zu ermitteln, und
**dass** die Ortsbestimmungs-Einrichtung (54) bevorzugt weiterhin ausgebildet ist, um die Höhe des landwirtschaftlichen Produkts (10) über dem Erdboden zu bestimmen.

6. Reifegrad-Bestimmungs-Vorrichtung (1) nach einem der Ansprüche 1 bis 5,
**dadurch gekennzeichnet,**
**dass** die Verarbeitungs-Einrichtung (50) ausgebildet ist, um anhand der Daten der Sensor-Einheit (2) einen optimalen Erntezeitpunkt des landwirtschaftlichen Produkts (10) zu ermitteln.

7. Reifegrad-Bestimmungs-Vorrichtung (1) nach einem der Ansprüche 1 bis 6,
**dadurch gekennzeichnet,**
**dass** die Reifegrad-Bestimmungs-Vorrichtung (1), bevorzugt die Sensor-Einheit (2) weiterhin eine Markierungs-Einrichtung (15, 46) aufweist, welche ausgebildet ist, um das landwirtschaftliche Produkt und/oder einen Bereich in der unmittelbaren Umgebung des landwirtschaftlichen Produkts (10) derart mit einer Markierung zu versehen, dass der Reifegrad des landwirtschaftlichen Produkts (10) und/oder ein optimaler Erntezeitpunkt aus der Markierung hervorgehen.

8. Reifegrad-Bestimmungs-Vorrichtung (1) nach Anspruch 7,
**dadurch gekennzeichnet,**
**dass** die Markierungs-Einrichtung (15, 46) bevorzugt ausgebildet ist, um eine Farbmarke und/oder eine Infrarot-Farbmarke und/oder eine Ultraviolett-Farbmarke und/oder eine chemischen Marke und/oder ein Ritzmuster und/oder eine Stanzung und/oder einen drahtlos auslesbaren Mikrochip, insbesondere ein RFID-Chip, auf das landwirtschaftliche Produkt (10) und/oder den Bereich in der unmittelbaren Umgebung des landwirtschaftlichen Produkts (10) aufzubringen.

9. Reifegrad-Bestimmungs-Vorrichtung (1) nach einem der Ansprüche 1 bis 8,
**dadurch gekennzeichnet,**
**dass** die Reifegrad-Bestimmungs-Vorrichtung (1) weiterhin eine Übertragungs-Einrichtung (21, 51) aufweist, welche ausgebildet ist, um sämtliche durch die Reifegrad-Bestimmungs-Vorrichtung (1) ermittelte Daten an eine Verarbeitungs-Einrichtung (60), welche nicht Teil der Reifegrad-Bestimmungs-Vorrichtung (1) ist zu übertragen.

10. Reifegrad-Bestimmungs-System (4) mit zumindest einer Reifegrad-Bestimmungs-Vorrichtung (1) nach einem der Ansprüche 1 bis 9, einer Verarbeitungs-Einrichtung (60) und einer Datenbank (65),
wobei die Reifegrad-Bestimmungs-Vorrichtung (1) ausgebildet ist, um ermittelte Daten an die Verarbeitungs-Einrichtung (60) zu übertragen,
wobei die Verarbeitungs-Einrichtung (60) ausgebildet ist, um die übermittelten Daten in der Datenbank (65) zu speichern.

11. Reifegrad-Bestimmungs-System (4) nach Anspruch 10,
**dadurch gekennzeichnet,**
**dass** das Reifegrad-Bestimmungs-System (4) weiterhin eine Anzeige-Einrichtung (64) aufweist, und
**dass** die Verarbeitungs-Einrichtung (60) ausgebildet ist, um in der Datenbank (65) gespeicherte Daten bezüglich des Reifegrads zumindest eines landwirtschaftlichen Produkts (10) auf der Anzeige-Einrichtung (64) grafisch darzustellen.

12. Reifegrad-Bestimmungs-System (4) nach Anspruch 11,
**dadurch gekennzeichnet,**
**dass** die Reifegrad-Bestimmungs-Vorrichtung (1) eine Reifegrad-Bestimmungs-Vorrichtung (1) nach Anspruch 5 ist, und
**dass** die Verarbeitungs-Einrichtung (60) ausgebildet ist, um in der Datenbank (65) gespeicherte Daten bezüglich des Reifegrads zumindest eines landwirtschaftlichen Produkts (10) gemeinsam mit dem geografischen Ort des landwirtschaftlichen Produkts (10) und vorzugsweise der Höhe über dem Erdboden des landwirtschaftlichen Produkts (10), vorzugsweise als Kartendarstellung, mittels der Anzeige-Einrichtung (64) grafisch darzustellen.

13. Reifegrad-Bestimmungs-System (4) nach einem der Ansprüche 10 bis 12,
**dadurch gekennzeichnet,**
**dass** das Reifegrad-Bestimmungs-System (4) weiterhin eine Steuereinrichtung (66) aufweist, welche ausgebildet ist, um zumindest ein weiteres damit verbundenes landwirtschaftliches Gerät zu steuern, und
**dass** das weitere landwirtschaftliche Gerät eine automatisierte Erntemaschine und/oder eine Bewässerungsanlage und/oder eine automatisierte Schädlingsbekämpfungsanlage und/oder eine automatisierte Düngungsanlage ist.

14. Reifegrad-Bestimmungs-System (4) nach einem der Ansprüche 10 bis 13,
**dadurch gekennzeichnet,**
**dass** das Reifegrad-Bestimmungs-System (4) weiterhin eine Ernteergebnis-Eingabe-Einrichtung aufweist, welche ausgebildet ist, um nach einer Ernte Daten, das Ernteergebnis betreffend, einzugeben, und
**dass** die Verarbeitungs-Einrichtung (60) ausgebildet ist, um eine Kalibrierung des Reifegrad-Bestimmungs-Systems (4) basierend auf dem eingegebenen Ernteergebnis und auf dem ermittelten Reifegrad zum Zeitpunkt der Ernte durchzuführen.

15. Verfahren zur Bestimmung eines Reifegrads eines landwirtschaftlichen Produkts (10),
wobei das Verfahren die folgenden Schritte beinhaltet:
- Bestimmung von Daten, welche auf den Reifegrad des landwirtschaftlichen Produkts (10) schließen lassen, vor der Ernte des landwirtschaftlichen Produkts (10), und
- Bestimmen des Reifegrads basierend auf den Daten, welche auf den Reifegrad des landwirtschaftlichen Produkts (10) schließen lassen.
